# EUROPEAN PATENT APPLICATION

(11) **EP 2 141 161 A1**
(43) Date of publication of application: **06.01.2010**
(21) Application number: 08159446.7
(22) Date of filing: 01.07.2008
(51) Int. Cl.: C07D 413/08, A61K 31/422, A61P 31/04

(54) **3-cyanopyrrolidinyl-phenyl-oxazolidinones as antibacterial agents**

(71) Applicant: Ferrer Internacional, S.A., 08028 Barcelona (ES)
(72) Inventor: CANO, Montserrat, 08691, Monistrol (Barcelona) (ES); PALOMER, Albert, 08014, Barcelona (ES); GUGLIETTA, Antonio, 08750, Molins de Rei (Barcelona) (ES)
(74) Representative: Barlocci, Anna

(57) **Abstract**

The invention provides new oxazolidinone compounds of formula (I) wherein R₁, R₂ and R₃ have different meanings.

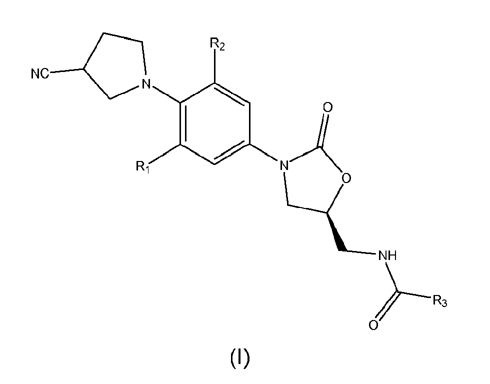

Preparative processes, pharmaceutical compositions, and uses thereof in the treatment of bacterial infections are also provided.

## Description

### TECHNICAL FIELD

This invention is directed to antimicrobial oxazolidinone compounds which are active against Gram-positive and some Gram-negative bacteria, showing specifically a potent activity against linezolid-resistant (LNZ-R) strains of Gram-positive bacteria and more specifically against Gram-positive pathogenic respiratory bacteria.

### BACKGROUND ART

Oxazolidinones are Gram-positive antimicrobial agents. Oxazolidinones bind to the 50S subunit of the prokaryotic ribosome, preventing formation of the initiation complex for protein synthesis. This is a novel mode of action. Other protein synthesis inhibitors either block polypeptide extension or cause misreading of mRNA. Linezolid (N-[[(5S)-3-[3-fluoro-4-(4-morpholinyl)phenyl]-2-oxo-5-oxazolidinyl]methyl]acetamide), US5688792, is the first approved antimicrobial oxazolidinone for clinical use in the United States and elsewhere. The structural formula of linezolid is: 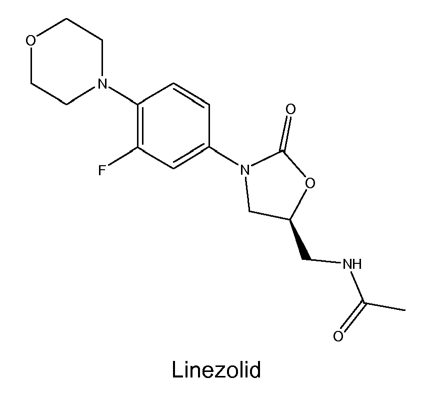 Linezolid minimal inhibitory concentrations (MICs) vary slightly with the test mode, laboratory, and significance attributed to thin hazes of bacterial survival, but all workers find that the susceptibility distributions are narrow and unimodal with MIC values between 0.5 and 4 µg/mL for *streptococci, enterococci* and *staphylococci.* Full activity is retained against Gram-positive *cocci* resistant to other antibiotics, including methicillin-resistant *staphylococci* and vancomycin-resistant *enterococci.* MICs are 2-8 µg/mL for *Moraxella, Pasteurella* and *Bacteroides* spp. but other Gram-negative bacteria are resistant as a result of endogenous efflux activity as well as the intake presented by Gram-negative bacteria outer membrane cell. Linezolid is indicated for the treatment of adult patients with the following infections: vancomycin-resistant *Enterococcus faecium* infections, including concurrent bacteremia; nosocomial pneumonia; complicated skin and skin structure infections; community-acquired pneumonia, including concurrent bacteremia; diabetic foot infections; and uncomplicated skin and skin structure infections.

Unfortunately, some Gram-positive bacteria such as *Staphylococcus aureus* (LNZ-R 432), *Haemophylus influenzae* (ATCC 49247), *Bacteroides fragilis* (ATCC 25285), *Moraxella catarrhalis* (HCl-78), and *Enterococcus faecium* (LNZ-R) show an important resistance to linezolid, thus suggesting the need of new oxazolidinone compounds active in these strains. Some of them are the origin of severe and sometimes fatal infections such as sepsis and septic shock. Further, there is an increasing need for improved agents against Gram-positive pathogenic respiratory bacteria, like *Streptococcus pneumoniae, Haemophylus influenzae*, and *Moraxella catarrhalis*.

### SUMMARY OF THE INVENTION

Surprisingly the compounds of the present application are potent active antimicrobial agents showing a relevant activity against LNZ-R Gram-positive bacteria and more specifically against Gram-positive pathogenic respiratory bacteria. Differential characteristic properties of the compounds of the present invention versus linezolid indicate the potential use thereof in severe infections that cannot be properly treated with linezolid.

In a first aspect the present invention refers to a compound of formula (I), 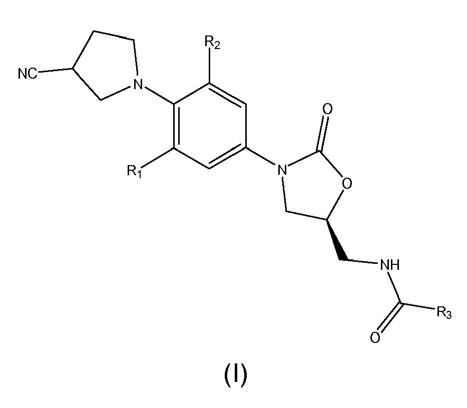 in free or pharmaceutically acceptable salt, solvate, hydrate, or enantiomeric form, wherein:
R₁ and R₂ are radicals identical or different and are independently selected from hydrogen and fluorine;
R₃ is a linear or branched (1-6C)alkyl group optionally substituted by a group selected from fluorine, hydroxy and OR₄; and
R₄ is a linear or branched (1-6C)alkyl group.

In a second aspect the present invention refers to a process for preparing a compound of formula (I) as defined in the first aspect of the invention in free or pharmaceutically acceptable salt, solvate, hydrate, or enantiomeric form that comprises:
(i) reacting an intermediate of formula (II), 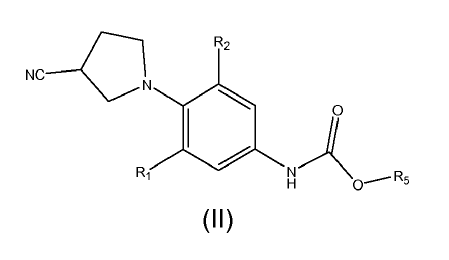 wherein R₁ and R₂ are as defined above and R₅ is selected from linear or branched (1-6C)alkyl and benzyl optionally phenyl-substituted by up to three linear or branched (1-6C)alkyl groups, with an intermediate of formula (III), 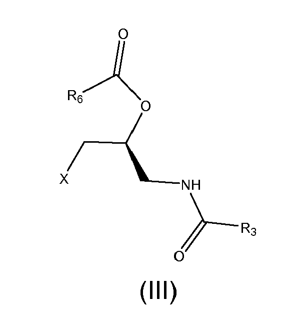 wherein R₃ is as defined above, R₆ is a linear or branched (1-6C)alkyl group, and X is a halogen atom; and
(ii) recovering the resultant compound of formula (I) in free or pharmaceutically acceptable salt, solvate, hydrate, or enantiomeric form.

In a third aspect the present invention refers to a pharmaceutical composition comprising a therapeutically effective amount of the compound of general formula (I) according to the first aspect of the invention, together with the appropriate amounts of pharmaceutical excipients or carriers.

In a fourth aspect the present invention refers to a compound of formula (I) according to the first aspect of the invention, for use as a medicament.

In an fifth aspect the present invention refers to the use of a compound of formula (I) according to the first aspect of the invention for the manufacture of a medicament for the treatment of bacterial infections in an animal or human. This aspect may also be formulated as a compound of formula (I) according to the first aspect of the invention for use in the treatment of bacterial infections.

Another object of this invention is to provide novel methods to treat a mammal, including a human, suffering from a bacterial infection by administering a therapeutically effective amount of a compound of formula (I) in free or pharmaceutically acceptable salt, solvate, hydrate, or enantiomeric form.

### DETAILED DESCRIPTION OF THE INVENTION

The term "pharmaceutically acceptable salts" used herein encompasses any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, tartaric acids, and the like, and any salt formed from organic and inorganic bases, such as the alkali metal and alkaline earth metal salts, especially the sodium and potassium salts, ammonium salts and salts of amines, including lower alkylated amines, such as methylamine, ethylamine, trimethylamine and the like, hydroxyloweralkylamines, such as ethanolamine and diethanolamine, and heterocyclic amines, such as morpholine and piperazine.

In a preferred embodiment, the present invention refers to a compound according to the first aspect of the invention wherein R₁ is fluorine, R₂ is selected from fluorine and hydrogen, and R₃ is methyl.

Preferably, the compound according to the first aspect of the invention is selected from the group consisting of:
*N*-{(5S)-3-[3-fluoro-4-(3-cyanopyrrolidin-1-yl)phenyl]-2-oxo-5-oxazolidinylmethyl}acetamide;
*N*-{(5*S*)-3-[3,5-difluoro-4-(3-cyanopyrrolidin-1-yl)phenyl]-2-oxo-5-oxazolidinylmethyl}acetamide;
*N*-{(5S)-3-[3-fluoro-4-(3(*R*)-cyanopyrrolidin-1-yl)phenyl]-2-oxo-5-oxazolidinylmethyl}acetamide;
*N*-{(5*S*)-3-[3,5-difluoro-4-(3(*R*)-cyanopyrrolidin-1-yl)phenyl]-2-oxo-5-oxazolidinylmethyl}acetamide;
*N*-{(5S)-3-[3-fluoro-4-(3(*S*)-cyanopyrrolidin-1-yl)phenyl]-2-oxo-5-oxazolidinylmethyl}acetamide; and
*N*-{(5*S*)-3-[3,5-difluoro-4-(3(*S*)-cyanopyrrolidin-1-yl)phenyl]-2-oxo-5-oxazolidinylmethyl}acetamide.

The compounds of general formula (I) may be prepared by
(i) reacting an intermediate of formula (II), 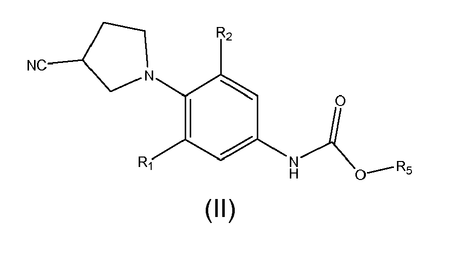 wherein R₁ and R₂ are as defined above and R₅ is selected from linear or branched (1-6C)alkyl and benzyl optionally phenyl-substituted by up to three linear or branched (1-6C)alkyl groups, with an intermediate of formula (III), 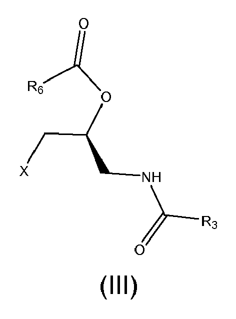 wherein R₃ is as defined above, R₆ is a linear or branched (1-6C)alkyl group, and X is a halogen atom, in an inert solvent and in the presence of a strong basic catalyst; and
(ii) recovering the resultant compound of formula (I) in free or pharmaceutically acceptable salt, solvate, hydrate, or enantiomeric form.

Preferably R₅ is benzyl, R₆ is methyl and X is bromine.

Inert solvents in step (i) are preferably aprotic solvents. Suitable aprotic solvents are polar ethers such as, for example, tetrahydrofuran, methyltetrahydrofuran, dioxane, *tert*-butylmethylether, or dimethoxyethylether, or amides such as, for example, dimethylformamide, or lactams such as, for example, N-methylpyrrolidone, and mixtures thereof. Suitable solvents are also mixtures of such aprotic solvents and alcohols such as, for example, methanol or ethanol.

Examples of strong basic catalysts include hydroxides such as lithium hydroxide, sodium hydroxide, and potassium hydroxide, alkoxides, such as lithium *tert*-butoxide, sodium *tert*-butoxide, and potassium *tert*-butoxide, alkyllithiums such as *tert*-butyllithium, n-butyllithium, and methyllithium, dialkylamides such as lithium diisopropylamide, disilylamides such as lithium hexamethyldisilazide, potassium hexamethyldisilazide, and sodium hexamethyldisilazide, and hydrides such as lithium hydride, sodium hydride, and potassium hydride.

Useful processes for recovering the resultant compounds in step (ii) include conventional methods known to the person skilled in the art such as crystallization and chromatographic processes, resolution of racemic forms by chromatographic separation using a chiral stationary phase, and also processes involving fractional crystallization. This can, in particular, involve the separation of individual enantiomers, for example, diastereoisomeric salts formed with chiral acids, for example (+)-tartaric acid, (-)-tartaric acid, or (+)-10-camphorsulfonic acid.

The compounds are useful antimicrobial agents, effective against a number of human and veterinary microorganisms. Some non limitative examples of these microorganisms are *Staphylococcus aureus, Streptococcus pneumoniae, Haemophylus influenzae, Bacteroides fragilis, Moraxella catarrhalis*, and *Enterococcus faecium*.

The compounds of the present invention can be normally formulated in accordance with standard pharmaceutical practice as a pharmaceutical composition.

The pharmaceutical compositions of this invention may be administered in standard manner for the disease condition that it is desired to treat, for example by oral, parenteral, inhalatory, rectal, transdermal or topical administration. For these purposes the compounds of this invention may be formulated by means known in the art in the form of, for example, tablets, capsules, syrups, aqueous or oily solutions or suspensions, emulsions, dispersible powders, inhalatory solutions, suppositories, ointments, creams, drops and sterile aqueous or oily solutions or suspensions for injection and the like. The pharmaceutical compositions may contain flavoring agents, sweeteners, etc. in suitable solid or liquid carriers or diluents, or in a suitable sterile media to form suspensions or solutions suitable for intravenous, subcutaneous or intramuscular injection. Such compositions typically contain from 1 to 40%, preferably 1 to 10% by weight of active compound, the remainder of the composition being pharmaceutically acceptable carriers, diluents, solvents and the like.

The compounds of formula (I) are administered in an amount of 0.1 to 100 mg/kg of body weight/day, preferably 1 to 50 mg/kg of body weight/day. The compounds and compositions of the present invention are useful in the treatment of conditions such as nosocomial pneumoniae, community acquired pneumoniae, caused by methicillin-resistant *Staphylococcus aureus* (MRSA), including concurrent bacteremia, penicillin resistance and sensitive *Streptococcus pneumoniae*, diabetic foot infections and skin and skin structure infections, and all other infections caused by bacteria sensitive to the compounds described in the invention. The compounds of the present invention are effective against a number of human or animal pathogens, clinical isolates, including vancomycin-resistant organisms, methicillin-resistant organisms, and LNZ-R organisms.

Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following examples are provided by way of illustration, and are not intended to be limiting of the present invention.

### EXAMPLES

### Example 1: N-{(5S)-3-[3-fluoro-4-(3-cyanopyrrolidin-1-yl)phenyl]-2-oxo-5-oxazolidinylmethy}acetamide

### a) 3-Fluoro-4-(3-cyanopyrrolidin-1-yl)nitrobenzene

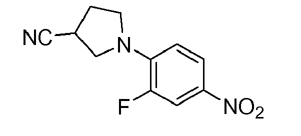

3-Carbonitrile pyrrolidine (133 mg) and potassium carbonate (268 mg) were dissolved in dimethylformamide (1 mL), and 3,4-difluoronitrobenzene (198 mg) added. The mixture was stirred at room temperature under nitrogen for 20 hours. Dichloromethane (DCM) was added to the reaction mixture and washed with water, brine and dried (magnesium sulfate). The residue was purified by column chromatography (11 g silica gel, DCM) to get 249 mg of title product (Yield = 80%).
HPLC (t, %): 8.04 min, 100%.
MS(ESI) *m*/*z* = 236 (M+1)
¹H NMR (400 MHz, , ppm, CDCl₃): 2.38 (2H, m), 3.27 (1 H, m), 3.67 (1 H, m), 3.77 (1 H, m), 3.89 (2H, m), 6.57 (1 H, t, J = 9.2 Hz), 7.92 (2H, m)

### b) 3-Fluoro-4-(3-cyanopyrrolidin-1-yl)phenylamine

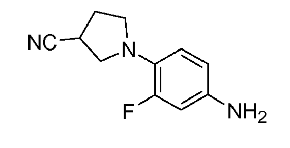

3-Fluoro-4-[3-cyanopyrrolidinyl]nitrobenzene (220 mg) was dissolved in ethanol (20 mL) and treated with tin chloride (SnCl₂.2H₂0, 1.5 g). The mixture was stirred and heated to reflux under nitrogen for 6 hours. Aqueous sodium bicarbonate and DCM were added, the organic layer separated and the aqueous layer extracted with DCM. The combined organic layers were dried and concentrated to give title product (141 mg. Yield = 73%).
HPLC (t, %): 6.41 min, 100%.

MS(ESI) *m*/*z* = 206 (M+1)
¹H NMR (400 MHz, , ppm, CDCl₃): 2.68 (2H, m), 3.21 (1H, m), 3.30 (2H, m), 3.35 (1 H, m), 3.49 (1 H, m), 6.45 (2H, m), 6.59 (1 H, t, J = 8 Hz)

### c) 3-Fluoro-4-(3-cyanopyrrolidin-1-yl)phenylcarbamic acid benzyl ester

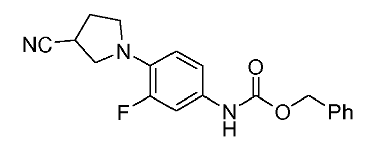

3-Fluoro-4-[3-cyanopyrrolidinyl]phenylamine (120 mg) was dissolved in acetone (4 mL) and cooled to 0°C. Sodium hydrogen carbonate (196 mg, 4 eq) in water (2 mL) was added, followed by benzyl chloroformate (199 mg, 2 eq) over 30 minutes. The mixture was stirred and the temperature allowed to rise to ambient over 3 hours. DCM was added and the organic layer separated, and washed with water and brine. The combined organic layers were dried over magnesium sulfate and concentrated. The residue was purified by column chromatography (10 gr silica gel) eluting with DCM and DCM/MeOH 98/2 to give 185 mg of title product (Yield = 93%).
HPLC (t, %): 9.06 min, 100%.
MS(ESI) *m*/*z* = 340 (M+1)
¹H NMR (400 MHz, , ppm, CDCl₃): 2.31 (2H, m), 3.17 (1H, m), 3.43 (2H, m), 3.54 (1 H, m), 3.67 (1 H, m), 5.17 (2H,s), 6.50 (1 H, m), 6.61 (1 H, t, J = 9 Hz), 6.90 (1 H, m), 7.38 (5H, m)

### d) N-{(5S)-3-[3-fluoro-4-(3-cyanopyrrolidin-1-yl)phenyl]-2-oxo-5-oxazolidinyl methyl}acetamide

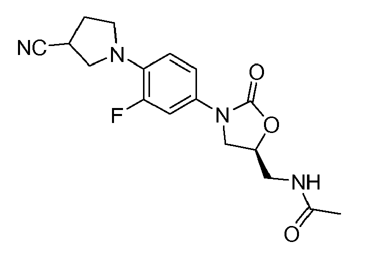

To a solution of 175 mg of 3-fluoro-4-[3-cyanopyrrolidinyl]-phenylcarbamic acid benzyl ester in 350 µL of dimethylformamide (DMF) was added 1.5 mL of a solution of 1M of lithium *tert*-butoxide in tetrahydrofuran (THF) and stirred at room temperature for 30 minutes. 42 µL of methanol and a solution of 246 mg of (*S*)-*N*-(3-bromo-2-acetoxypropyl)acetamide in 350 µL of DMF were added and allowed to stand at room temperature for two days at which point HPLC showed a 30% conversion. The same amount of lithium *tert*-butoxide in THF, methanol and (*S*)-*N*-(3-bromo-2-acetoxypropyl)acetamide were added and reaction mixture stirred at room temperature for two days more. Saturated aqueous ammonium chloride was added to the reaction solution and the separated organic layer was washed with water, brine and dried over anhydrous magnesium sulfate. The solvent was evaporated and the residue was purified by silica gel column chromatography (DCM, methanol in increasing polarity) to afford 113 mg of the title compound (Yield = 63%).
HPLC (t, %): 6.58 min, 100%.
MS(ESI) *m*/*z* = 347 (M+1)
¹H NMR (400 MHz, , ppm, DMSO): 2.16 (1H, m), 2.30 (1H, m), 3.38 (4H, m), 3.66 (1H, dd, J = 6.8, 8.8 Hz), 4.05 (1H, t, J = 9 Hz), 4.67 (1H, m), 6.83 (1H, t, J = 9 Hz), 7.11 (1H, dd, J = 2.4, 9 Hz), 7.43 (1H, dd, J = 2.8, 16 Hz)

### Example 2: N-{(5S)-3-[3,5-difluoro-4-(3-cyanopyrrolidin-1-yl)phenyl]-2-oxo-5-oxazolidinylmethyl}acetamide

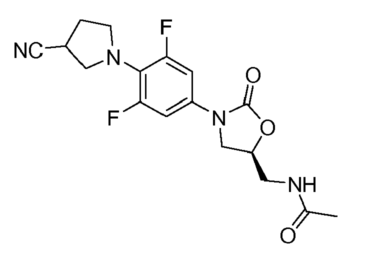

It was obtained in a similar way than compound of Example 1, starting from alkylation of 3, 4, 5-trifluoronitrobenzene with 3-carbonitrile pyrrolidine.
HPLC (t, %): 6.94 min, 92%.
MS(ESI) *m*/*z* = 365 (M+1)
¹H NMR (400 MHz, , ppm, CDCl₃): 1.82 (3H, s), 2.25 (2H, m), 3.13 (1H, q, J = 7.2 Hz), 3.58 (7H, m), 3.96 (1H, t, J = 9.2 Hz), 4.75 (1H, m), 6.04 (1H, NH), 7.06 (2H, d, J = 10.8 Hz)

### Example 3: Pharmaceutical compositions

The following illustrate representative pharmaceutical compositions containing a compound of formula (I) or a pharmaceutically acceptable salt thereof for antimicrobial use in humans or animals:

| Tablet 1 | mg/tablet |
|---|---|
| Active ingredient | 100 |
| Lactose | 179 |
| Croscarmellose sodium | 12 |
| Polyvinylpyrrolidone | 6 |
| Magnesium stearate | 3 |

| Tablet 2 | mg/tablet |
|---|---|
| Active ingredient | 50 |
| Lactose | 229 |
| Croscarmellose sodium | 12 |
| Polyvinylpyrrolidone | 6 |
| Magnesium stearate | 3 |

| Tablet 3 | mg/tablet |
|---|---|
| Active ingredient | 1 |
| Lactose | 92 |
| Croscarmellose sodium | 4 |
| Polyvinylpyrrolidone | 2 |
| Magnesium stearate | 1 |

| Capsule | mg/capsule |
|---|---|
| Active ingredient | 10 |
| Lactose | 389 |
| Croscarmellose sodium | 100 |
| Magnesium stearate | 1 |

| Injection | 50 mg/mL |
|---|---|
| Active ingredient | 5.0% w/v |
| Isotonic aqueous solution | to 100% |

Buffers, pharmaceutically acceptable co-solvents such as polyethylene glycol, polypropylene glycol, glycerol or ethanol or complexing agents, may be used to aid formulation.

The above formulations may be prepared by well-known conventional procedures in the pharmaceutical art. The tablets 1-3 may be enteric coated by conventional means, for example to provide a coating of cellulose acetate phthalate.

### Example 4: Antibacterial activity

MICs were determined by using a standard micro dilution method according to The National Committee for Clinical Laboratory Standards (NCCLS), 5^{th} Approved standard M7-A5, 2001, Wayne, PA, USA. All compounds were tested against Gram-positive and Gram-negative bacteria showing relevant different susceptibility and resistance specifications. The used micro organisms were selected from laboratory reference bacteria and from clinical isolates.The tested concentrations were double dilutions from 0.06 µg/mL to 128 µg/mL in 96-well micro titter plates.

MICs were determined in the *Brucella* Blood medium supplemented for the anaerobic strains, and in the Mueller-Hinton culture medium (cation-adjusted) for the aerobic bacteria.

The tested compounds were dissolved in DMSO, and were diluted as far as 2560 µg/mL with the different media according to the specific requirements for each group of strains. The 96-well sealed micro titter plates containing bacteria were incubated in different laboratory conditions depending on the nature of the microorganism. Thus, the aerobic bacteria were incubated during 16-24 h at 35°C and the so-called fastidious bacteria, such as *M. catarrhalis* and *S. pneumoniae*, during 20-24h at 35°C in a microaerobiotic atmosphere containing 5% CO₂ (Anaerocult C, MERCK). The results of these tests are given in Table 1.

**Table 1**

| | Ex. 1 | Ex. 2 | Linezolid |
|---|---|---|---|
| *S. aureus* ATCC25923 MS | 0.5 | 0.25 | 1.00 |
| *S. pneumoniae* ATCC49619 PR | 0.25 | 0.125 | 1.00 |
| *E. faecium* ATCC51559 MDR | 0.25 | 0.125 | 0.50 |
| *S. aureus* LNZ-R 432 | 4 | 64 | 16-32 |
| *H. influenzae* ATCC49247 | 4 | 2 | 16.00 |
| *B. fragilis* sp. fragilis ATCC25285 | 0.5 | 0.125 | 2.00 |
| *Moraxella* *catarrhalis* HCl-78 | 2 | 0.25 | 2.00 |
| *E. faecium* LNZ-R LR-4 | 8 | 4 | 64.00 |

## Claims

1. A compound of formula (I), 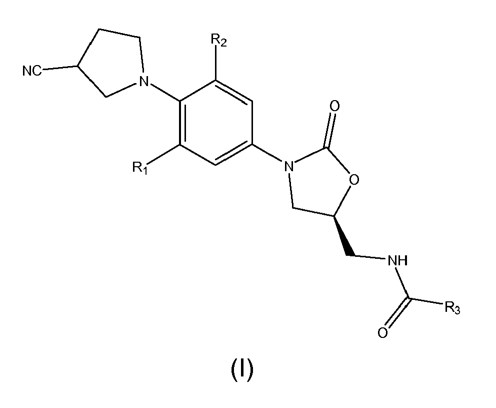 in free or pharmaceutically acceptable salt, solvate, hydrate, or enantiomeric form, wherein:
R₁ and R₂ are radicals identical or different and are independently selected from hydrogen and fluorine;
R₃ is a linear or branched (1-6C)alkyl group optionally substituted by a group selected from fluorine, hydroxy and OR₄; and
R₄ is a linear or branched (1-6C)alkyl group.

2. The compound according to claim 1, wherein R₁ is fluorine.

3. The compound according to claim 1, wherein R₂ is selected from fluorine and hydrogen.

4. The compound according to claim 1, wherein R₃ is methyl.

5. The compound as claimed in any of the preceding claims, which is selected from the group consisting of:
*N*-{(5S)-3-[3-fluoro-4-(3-cyanopyrrolidin-1-yl)phenyl]-2-oxo-5-oxazolidinylmethyl}acetamide;
*N*-{(5*S*)-3-[3,5-difluoro-4-(3-cyanopyrrolidin-1-yl)phenyl]-2-oxo-5-oxazolidinylmethyl}acetamide;
*N*-{(5S)-3-[3-fluoro-4-(3(*R*)-cyanopyrrolidin-1-yl)phenyl]-2-oxo-5-oxazolidinylmethyl}acetamide;
*N*-{(5*S*)-3-[3,5-difluoro-4-(3(*R*)-cyanopyrrolidin-1-yl)phenyl]-2-oxo-5-oxazolidinylmethyl}acetamide;
*N*-{(5S)-3-[3-fluoro-4-(3(S)-cyanopyrrolidin-1-yl)phenyl]-2-oxo-5-oxazolidinylmethyl}acetamide; and
*N*-{(5*S*)-3-[3,5-difluoro-4-(3(*S*)-cyanopyrrolidin-1-yl)phenyl]-2-oxo-5-oxazolidinylmethyl}acetamide.

6. A process for preparing a compound of formula (I) in free or pharmaceutically acceptable salt, solvate, hydrate, or enantiomeric form that comprises:
(i) reacting an intermediate of formula (II), 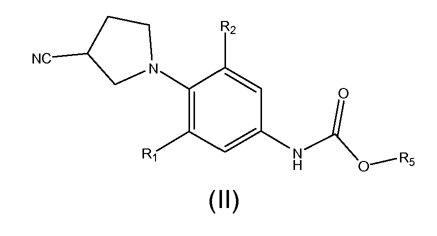 wherein R₁ and R₂ are as defined above and R₅ is selected from linear or branched (1-6C)alkyl and benzyl optionally phenyl-substituted by up to three linear or branched (1-6C)alkyl groups, with an intermediate of formula (III), 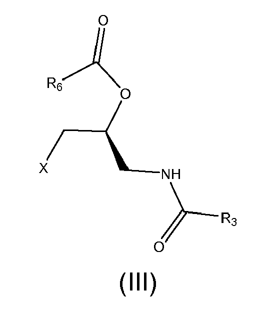 wherein R₃ is as defined above, R₆ is a linear or branched (1-6C)alkyl group, and X is a halogen atom; and
(ii) recovering the resultant compound of formula (I) in free or pharmaceutically acceptable salt, solvate, hydrate, or enantiomeric form.

7. The process of claim 6 wherein R₅ is benzyl, R₆ is methyl and X is bromine.

8. A pharmaceutical composition comprising a therapeutically effective amount of the compound of formula (I) as defined in any of the claims 1 to 5, together with the appropriate amounts of pharmaceutical excipients or carriers.

9. A compound of formula (I) as defined in any of the claims 1 to 5 for use as a medicament.

10. Use of a compound of formula (I) as defined in any of the claims 1 to 5 for the manufacture of a medicament for the treatment of bacterial infections in an animal or human.

11. A compound of formula (I) as defined in any of the claims 1 to 5 for use in the treatment of bacterial infections.
